(19) Europäisches Patentamt
European Patent Office
Office européen des brevets

(11) Veröffentlichungsnummer : **0 149 208**
**B1**

(12) # EUROPÄISCHE PATENTSCHRIFT

(45) Veröffentlichungstag der Patentschrift : **04.05.88**

(21) Anmeldenummer : **84116076.5**

(22) Anmeldetag : **21.12.84**

(51) Int. Cl.⁴ : **C 07 C121/46**, C 07 C121/60, C 07 D239/26, C 07 D241/12, C 09 K 19/00

(54) **Ethanderivate.**

(30) Priorität : **17.01.84 DE 3401320**

(43) Veröffentlichungstag der Anmeldung : **24.07.85 Patentblatt 85/30**

(45) Bekanntmachung des Hinweises auf die Patenterteilung : **04.05.88 Patentblatt 88/18**

(84) Benannte Vertragsstaaten : **CH DE FR GB LI**

(56) Entgegenhaltungen :
**EP-A- 0 056 501**
**EP-A- 0 058 981**
**EP-A- 0 084 194**
**EP-A- 0 097 033**
**EP-A- 0 125 563**
**EP-A- 0 129 177**
**DE-A- 3 332 690**
**GB-A- 2 092 169**

(73) Patentinhaber : **Merck Patent Gesellschaft mit beschränkter Haftung**
**Frankfurter Strasse 250**
**D-6100 Darmstadt (DE)**

(72) Erfinder : **Fearon, Julie Elizabeth**
**Flat 3, 91 Westbourne Avenue**
**Hull HU3 5BS (GB)**
Erfinder : **Smith, Wendy Elaine**
**"Lynwode House" 207 Lincoln Road**
**Branston Lincoln (GB)**
Erfinder : **Gray, George William**
**"Glenwood" Newgate Street**
**Cottingham HU16 4DY (GB)**
Erfinder : **Lacey, David, Dr.**
**19 Kildale Close**
**Hull HU8 9NW (GB)**
Erfinder : **Toyne, Kenneth Johnson, Dr.**
**25 Hall Road**
**Hull HU6 8QW (GB)**
Erfinder : **Weber, Georg**
**Wilhelm Leuschner-Strasse 38**
**D-6106 Erzhausen (DE)**

**EP 0 149 208 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Ethanderivate der Formel I

$$R^1—A^1—CH_2CH_2—A^2—(A^3)_n—R^2 \qquad I$$

worin

$R^1$ H, eine Alkylgruppe mit 1 - 12 C-Atomen, worin auch eine oder zwei nicht benachbarte $CH_2$-Gruppen durch O-Atome und/oder CO-Gruppen und/oder -O-CO-Gruppen und/oder -CO-O-Gruppen ersetzt sein können, F, C1, Br

$R^2$ —CN oder $—Z^2— {}^4—CN$

$A^1$ —A—, $—A—A^5—$ oder $—A^5—A—$,

$A^2$ eine unsubstituierte oder durch ein oder zwei F— und/oder C1-Atome und/oder $CH_3$-Gruppen und/oder CN-Gruppen substituierte 1,4-Phenylen-Gruppe, worin auch eine oder zwei CH-Gruppen durch N-Atome ersetzt sein können,

A eine 1,4-Cyclohexylen-Gruppe, worin auch eine oder zwei nicht benachbarte $CH_2$-Gruppen durch O-Atome ersetzt sein können, eine 1,3-Dithian-2,5-diylgruppe oder eine 1,4-Bicyclo(2,2,2)-octylen-Gruppe,

$A^3$, $A^4$ und $A^5$ jeweils eine 1,4-Cyclohexylengruppe, worin auch eine oder zwei nicht benachbarte $CH_2$-Gruppen durch O-Atome ersetzt sein können, eine 1,3-Dithian-2,5-diylgruppe, eine unsubstituierte oder durch ein oder zwei F- und/oder C1-Atome und/oder $CH_3$-Gruppen und/oder CN-Gruppen substituierte 1,4-Phenylen-Gruppe, worin auch eine oder zwei CH-Gruppen durch N-Atome ersetzt sein können,

n O oder 1,

$Z^2$ $—CH_2CH_2—$, $—CH_2O—$, $—OCH_2—$, —O—CO— oder eine Einfachbindung, und

bedeutet, mit der Maßgabe, daß

a) im Falle $A^2 = A^3 =$ 1,4-Phenylen (n = 1) und/oder im Falle $R^1—A^1$ = p-(trans-4-Alkylcyclohexyl)-phenyl mindestens eine der Gruppen $A^2$ bzw. $A^3$ lateral substituiert ist, und

b) im Falle $A^1$ = A und/oder $R^1—A^1$ = trans-4-(trans-4-Alkylcyclohexyl)-cyclohexyl n 1 und $A^3$ eine unsubstituierte oder durch ein oder zwei F- und/oder C1-Atome und/oder $CH_3$-Gruppen und/oder CN-Gruppen substituierte 1,4-Phenylen-gruppe bedeutet, und

c) p-[2-[trans-4-(p-Alkylphenyl oder p-Alkoxyphenyl)cyclohexyl]-äthyl]benzonitrile ausgeschlossen sind.

Der Einfachheit halber bedeuten im folgenden Phe eine 1,4-Phenylengruppe, Cy eine 1,4-Cyclohexylengruppe, Dio eine 1,3-Dioxan-2,5-diylgruppe, Dit eine 1,3-Dithian-2,5-diylgruppe, Bi eine Bicyclo(2,2,2)-octylengruppe, Pip eine Piperidin-1,4-diylgruppe, Pyn eine Pyridazin-3,6-diylgruppe und Pyr eine Pyrimidin-2,5-diylgruppe, wobei diese Gruppen, insbesondere die 1,4-Phenylengruppe, unsubstituiert oder durch ein oder zwei F- und/oder C1-Atome und/oder $CH_3$-Gruppen und/oder CN-Gruppen substituiert sein können.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

In EP-A-0 097 033 werden heterocyclische Ethanderivate beschrieben, die jedoch wesentlich niedrigere Klärpunkte zeigen als die Verbindungen der Formel I. In den Dokumenten GB-A-2 092 169 und EP-A-0 056 501 werden spezielle Flüssigkristallmischungen mit bekannten Komponenten beschrieben, die sich für Zellen im Multiplexbetrieb eignen. Für die dielektrisch positiven Komponenten dieser Mischungen ist eine sehr breite allgemeine Formel angegeben, die teilweise auch die Ethanderivate der Formel I umfaßt. Keine der beschriebenen Mischungen enthält jedoch ein Ethanderivat der Formel I. Flüssigkristallverbindungen mit ähnlicher Struktur sind in EP-A-0 125 563 (trans,trans-4-[2-p-(trans-4-Cyancyclohexyl)-phenyl-ethyl]-4'-alkylbicyclohexyl) und EP-A-0 129 177 (p-[2-[trans-4-(p-Alkylphenyl und p-Alkoxyphenyl)-cyclohexyl]-äthyl]benzonitrile) beschrieben.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit kleinen $k_{33}/k_{11}$-Werten, sehr niedriger Viskosität und vergleichsweise kleiner optischer Anisotropie herstellbar. Verbindungen der Formel I sind ferner teilweise als Komponenten für smektische Phasen (zum Beispiel für den Speichereffekt) geeignet.

Mit der Bereitstellung der Verbindungen der Formel I wird außerdem ganz allgemein die Palette der flüssigkristallinen Substanzen, die nich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind ; es können aber auch

Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man ein entsprechendes Carbonsäureamid dehydratisiert oder ein entsprechendes Carbonsäurehalogenid mit Sulfamid umsetzt, oder daß man eine entsprechende Brom- oder Chlorverbindung mit einem Cyanid umsetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Vor- und nachstehend haben $R^1$, $R^2$, $A^1$, $A^2$, $A^3$, $A^4$, $A^5$, A und $Z^2$ die angegebene Bedeutung, sofern nicht ausdrücklich etwas anderes vermerkt ist.

Die Verbindungen der Formel I umfassen dementsprechend Verbindungen der Teilformeln Ib ist Id (mit jeweils drei Ringen) sowie Ig bis Ih (mit vier Ringen) :

| | |
|---|---|
| $R^1$—A—$A^5$—$CH_2CH_2$—$A^2$—CN | Ib |
| $R^1$—$A^5$—A—$CH_2CH_2$—$A^2$—CN | Ic |
| $R^1$—A—$CH_2CH_2$—$A^2$—$A^3$—CN | Id |
| $R^1$—A—$A^5$—$CH_2CH_2$—$A^2$—$A^3$—CN | Ig |
| $R^1$—$A^5$—A—$CH_2CH_2$—$A^2$—$A^3$—CN | Ih |

Die bevorzugten Verbindungen der Teilformel Ib umfassen solche der Teilformeln Iba bis Ibc :

| | |
|---|---|
| $R^1$—Cy—Phe—$CH_2CH_2$—PhX—CN | Iba |
| $R^1$—Dio—Phe—$CH_2CH_2$—PhX—CN | Ibb |
| $R^1$—Dit—Phe—$CH_2CH_2$—PhX—CN | Ibc |

Darunter sind diejenigen der Teilformel Iba besonders bevorzugt.

In den vor- und nachstehenden Formeln bedeutet PhX eine durch ein- oder zwei F- und/oder C1-Atome und/oder $CH_3$-Gruppen und/oder CN-Gruppen substituierte 1,4-Phenylengruppe.

Die bevorzugten Verbindungen der Teilformel Id um- fassen solche der Teilformeln Ida bis Idi :

| | |
|---|---|
| $R^1$—A—$CH^2CH^2$—Pyr—Phe—CN | Ida |
| $R^1$—A—$CH_2CH_2$—Pyr—PhX—CN | Idb |
| $R^1$—A—$CH_2CH_2$—PhX—Phe—CN | Idc |
| $R^1$—A—$CH_2CH_2$—Phe—PhX—CN | Ide |
| $R^1$—A—$CH_2CH_2$—PhX—Cy—CN | Idf |
| $R^1$—A—$CH_2CH_2$—Pyn—Phe—CN | Idg |
| $R^1$—A—$CH_2CH_2$—Pyn—Cy—CN | Idh |
| $R^1$—A—$CH_2CH_2$—Phe—Cy—CN | Idi |

Darunter sind diejenigen der Teilformeln Ida bis Idf, insbesondere Idb bis Ide, besonders bevorzugt. Weiterhin bevorzugt ist Idi. Besonders bevorzugt sind Verbindungen der Formeln Idc und Ide worin A trans-1,4-Cyclohexylen und $R^1$ Alkyl mit 1-12 C-Atomen bedeuten.

Die bevorzugten Verbindungen der Teilformel Ig umfassen solche der Teilformeln Iga bis Igh :

| | |
|---|---|
| $R^1$—A—Phe—$CH_2CH_2$—PhX—Phe—CN | Iga |
| $R^1$—A—Phe—$CH_2CH_2$—Phe—PhX—CN | Igb |
| $R^1$—A—Phe—$CH_2CH_2$—Phe—PhX—CN | Igc |
| $R^1$—A—Phe—$CH_2CH_2$—PhX—Cy—CN | Igd |
| $R^1$—A—PhX—$CH_2CH_2$—Phe—Cy—CN | Ige |
| $R^1$—A—Phe—$CH_2CH_2$—Pyr—Phe—CN | Igf |
| $R^1$—A—Phe—$CH_2CH_2$—Pyn—Phe—CN | Igh |

Darunter sind diejenigen der Teilformeln Iga bis Igc besonders bevorzugt.

Die bevorzugten Verbindungen der Teilformel Ih umfassen solche der Teilformeln Iha bis Ihf :

| | |
|---|---|
| $R^1$—Cy—A—$CH_2CH_2$—PhX—PHe—CN | Iha |
| $R^1$—Cy—A—$CH_2CH_2$—Phe—PhX—CN | Ihb |
| $R^1$—Cy—A—$CH_2CH_2$—PhX—Cy—CN | Ihc |

| | |
|---|---|
| $R^1—Cy—A—CH_2CH_2—Pyr—Phe—CN$ | Ihd |
| $R^1—Cy—A—CH_2CH_2—Pyn—Phe—CN$ | Ihe |
| $R^1—Cy—A—CH_2CH_2—Phe—Cy—CN$ | Ihf |

Darunter sind diejenigen der Teilformeln Iha und Ihb besonders bevorzugt. Weiterhin bevorzugt ist Ihf.

In den Verbindungen der vor- und nachstehenden Formeln bedeutet $R^1$ vorzugsweise Alkyl, ferner Alkoxy oder eine andere Oxaalk lgruppe. $R^2$ ist bevorzugt —CN.

$A^1$ ist bevorzugt —A— oder —A—$A^5$.

A ist bevorzugt Cy, Dio oder Dit, insbesondere Cy.

$A^2$ ist bevorzugt Ph, PhX, Pyr, Pyn oder eine Pyridin-2,5-diylgruppe, insbesondere Ph oder PhX.

PhX ist bevorzugt eine in 2- oder 3-Stellung durch F, Cl oder $CH_3$, insbesondere F oder $CH_3$, substituierte 1,4-Phenylengruppe.

$A^3$, $A^4$ und $A^5$ sind bevorzugt Ph, PhX, Cy, Dio, ferner bevorzugt Dit, Pyr oder Pyn.

$A^3$ und $A^4$ sind bevorzugt Ph und Cy.

Bevorzugt enthält die Verbindung der Formel I nicht mehr als einen der Reste Dio, Dit, PhX, Pyr oder Pyn.

$Z^2$ ist bevorzugt eine Einfachbindung, in zweiter Linie bevorzugt eine —O—CO— oder —$CH_2$—$CH_2$-Gruppe.

Falls $R^1$ einen Alkylrest bedeutet, in dem auch eine (« Alkoxy » bzw. « Oxaalkyl ») oder zwei (« Alkoxyalkoxy » bzw. « Dioxaalkyl ») nicht benachbarte $CH_2$-Gruppen durch O-Atome ersetzt sein können, so kann er geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formel I mit verzweigter Flügelgruppe $R^1$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Chirale Verbindungen der Formel I sind weiterhin zur Herstellung smektischer $S_c$ *-Phasen geeignet. Verzweigte Gruppen dieser Art enthalten in der Regel nicht mehr als eine Kettenverzweigung. Bevorzugte verzweigte Reste $R^1$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Octyloxy, 2-Oxa-3-methylbutyl, 3-Oxa-4-methylpentyl.

Unter den Verbindungen der Formel I sind diejenigen bevorzugt, in denen mindestens einer der darin enthaltenen Reste eine der angegebenen bevorzugten Bedeutungen hat. Weiterhin bevorzugt sind Verbindungen der Formel I, worin $A^2$ oder $A^3$, insbesondere $A^3$, eine durch ein oder zwei F- und/oder C1-Atome und/oder $CH_3$-Gruppen und/oder CN-Gruppen, insbesondere durch ein oder zwei F-Atome oder $CH_3$-Gruppen, substituierte 1,4-Phenylengruppe bedeutet, sowie Verbindungen der Formel I, worin A eine 1,4-Cyclohexylen-Gruppe bedeutet.

Unter den Verbindungen der Formel I sind diejenigen Stereoisomeren bevorzugt, in denen die cycloaliphatischen Reste (z. B. Cy, Dio, Dit) trans-1,4-disubstituiert sind.

Diejenigen der vorstehend genannten Formeln, die eine oder mehrere der Gruppen Dio, Dit und/oder Pyr enthalten, umschließen jeweils die beiden möglichen 2,5-Stellungsisomeren.

Besonders bevorzugt sind Verbindungen der Formel I sowie der vorstehenden Teilformeln, worin $R^1$ eine geradkettige Alkylgruppe oder Alkoxygruppe mit jeweils 1 bis 10 C-Atomen bedeutet. Weiterhin bevorzugt sind Verbindungen der Formel I, worin mindestens eine der Gruppen $A^2$, $A^3$, $A^4$ und $A^5$ Pyr, Pyn oder Pyrazin-2,5-diyl, insbesondere Pyr, bedeutet (d. h. 1,4-Phenylen, worin zwei CH-Gruppen durch N-Atome ersetzt sind).

Die Verbindungen der Formel I werden nach an sich bekannten Methoden hergestellt, wie sie in der Literatur (z. B. in den Standardwerken wie Houben-Weyl, Methoden der Organischen Chemie, Georg Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der Fachmann kann durch Routinemethoden entsprechende Synthesemethoden aus dem Stand der Technik entnehmen (z. B. DE-OS 23 44 732, 24 50 088, 24 29 093, 25 02 904, 26 36 684, 27 01 591 und 27 52 975 betreffend Verbindungen mit 1,4-Cyclohexylen- und 1,4-Phenylen-Gruppen ; DE-PS 26 41 724 betreffend Verbindungen mit Pyrimidin-2,5-diyl-Gruppen ; DE-OS 32 28 350 betreffend Verbindungen mit Pyridazin-3,6-diyl-Gruppen ; JP-OS 58-43 961 betreffend Verbindungen mit Pyrazin-2,5-diyl-Gruppen ; DE-OS 29 44 905 und 32 27 916 betreffend Verbindungen mit 1,3-Dioxan-2,5-diyl-Gruppen ; DD 160 061 betreffend Verbindungen mit 1,3-Dithian-2,5-diyl-Gruppen ; US 4,261,652 und 4,219,256 betreffend

Verbindungen mit 1,4-Bicyclo(2,2,2)-octylen-Gruppen ; DE-OS 31 50 312 betreffend Verbindungen mit Decahydronaphthalin-2,6-diyl-Gruppen ; DE-OS 29 49 080 betreffend Verbindungen mit 1,2,3,4-Tetrahydronaphthalin-2,6-diyl-Gruppen ; DE-OS 32 08 089, DE-OS 31 17 152, DE-OS 30 42 391 und EP-OS 84 194 betreffend lateral substituierter Verbindungen ; und DE-OS 32 01 721 betreffend Verbindungen mit —$CH_2$—$CH_2$-Brückengliedern).

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergstellt werden, indem man ein entsprechendes Carbonsäureamid dehydratisiert oder ein entsprechendes Carbonsäurehalogenid mit Sulfamid umsetzt. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten ; als Lösungsmittel kommen z. B. Basen wie Pyridin oder Triethylamin, aromatische Kohlenwasserstoffe wie Benzol, Toluol oder Xylol oder Amide wie DMF in Betracht.

Zur Herstellung der Ethanderivate der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Für die Ausgangsstoffe kann der Fachmann entsprechende Synthesemethoden aus dem Stand der Technik entnehmen. Benzoesäurehalogenide (entsprechend Formel I, worin —$A^2$—$R^2$ = —Ph-COHalogen, —$A^2$—Ph-COHalogen, —$A^3$—$Z^2$-Ph-COHalogen, —PhX-COHalogen, —$A^2$PhX-COHalogen oder —$A^3$—$Z^2$-PhX-COHalogen) können beispielsweise aus entsprechenden Benzolderivaten (z. B. solche, in denen an Stelle des Restes COHalogen ein H-Atom steht) durch Umsetzung mit Oxalylhalogenid, vorzugsweise Oxalylchlorid, unter Friedel-Crafts-Bedingungen (GB-Patentanmeldung 8 206 265) hergestellt werden.

Entsprechende Cyclohexancarbonsäurederivate (z. B. Säurehalogenide, Ester ; entsprechend Formel I, worin —$A^2$—$R^2$ beispielsweise —$A^3$—Cy—CN bedeutet) sind beispielsweise aus Halogenverbindungen (vorzugsweise Bromiden oder Chloriden) entsprechend Formel I, worin —$A^2$—$R^2$ —$A^3$-Halogen bedeutet, zugänglich durch Grignard- Reaktion mit 4-(4,5-Dihydro-4,4-dimethyl-2-oxazolyl)-cyclohexanon, Abspaltung von Wasser unter gleichzeitiger Entfernung der Oxazolylschutzgruppe durch Kochen mit ethanolischer $H_2SO_4$, Hydrierung der Doppelbindung und ggf. Überführung des Ethylesters in das gewünschte Säurederivat nach literaturbekannten Methoden.

Zur Herstellung von Ethanderivaten der Formel I können auch entsprechende Chlor- oder Bromverbindungen nach literaturbekannten Methoden mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zweichen 20° und 200°.

Für die Chlor- oder Bromverbindungen kann der Fachmann entsprechende Synthesemethoden aus dem Stand der Technik entnehmen. Beispielsweise sind diese Verbindungen durch Halogenierung der oben beschriebenen Benzolderivate zugänglich.

Die erfindungsgemäßen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Diphenylethane, 1,2-Dicyclohexylethane, 1-Cyclohexyl-2-phenylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

$$R'—L—G—E—R'' \qquad II$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | —CH=CH— | —N(O)=N— |
| | —CH=CY— | —CH=N(O)— |
| | —C≡C— | —$CH_2$—$CH_2$— |
| | —CO—O— | —$CH_2$—O— |

—CO—S— —$CH_2$—S—
—CH=N— —COO—Phe—COO—

oder eine C-C-Einfachbindung, Y Halogen, vorzugsweise Chlor, oder —CN, und R' und R" Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R" voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle dieser Substanzen sind nach literaturbekannten Methoden herstellbar.

Die erfindungsgemäßen Phasen enthalten etwa 0,1 bis 99, vorzugsweise 10 bis 95 %, einer oder mehrerer Verbindungen der Formel I.

Weiterhin bevorzugt sind erfindungsgemäße Dielektrika, enthaltend 0,1 bis 40, vorzugsweise 0,5 bis 30 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z. B. I. Haller et al., Mol. Cryst. Liq. Cryst. Band 24, Seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent ; alle Temperaturangaben sind in Grad Celsius angegeben. « Übliche Aufarbeitung » bedeutet : man gibt Wasser hinzu, extrahiert mit Methylenchlorid, trennt ab, trocknet die organische Phase, dampft ein und reinigt das Produkt durch Kristallisation und/oder Chromatographie.

## Beispiel 1

36,7 m 1-[4-(trans-4-n-Propylcyclohexyl)-phenyl]-2-(4-carbamoyl-3-fluor-phenyl)-ethan werden in 200 ml Methylenchlorid suspendiert und nach Zugabe von 20 g Thionylchlorid und 3,5 ml Dimethylformamid 4 Stunden gekocht. Nach dem Abkühlen versetzt man mit 500 ml Eiswasser, trennt die organische Phase ab und arbeitet wie üblich auf. Nach Umkristallisation aus Methanol erhält man 18,1 g (52 %) 1-[4-(trans-4-n-Propylcyclohexyl)-phenyl]-2-(4-cyan-3-fluorphenyl)-ethan.

Analog werden hergestellt :

1-[p-(trans-4-Ethylcyclohexyl)-phenyl]-2-(4-cyan-3-fluor-phenyl)-ethan
1-[p-(trans-4-Butylcyclohexyl)-phenyl]-2-(4-cyan-3-fluor-phenyl)-ethan
1-[p-(trans-4-Pentylcyclohexyl)-phenyl]-2-(4-cyan-3-fluor-phenyl)-ethan
1-[p-(trans-4-Heptylcyclohexyl)-phenyl]-2-(4-cyan-3-fluor-phenyl)-ethan
1-[p-(trans-4-Ethylcyclohexyl)-phenyl]-2-(4-cyan-3-methyl-phenyl)-ethan
1-[p-(trans-4-Propylcyclohexyl)-phenyl]-2-(4-cyan-3-methyl-phenyl)-ethan
1-[p-(trans-4-Butylcyclohexyl)-phenyl]-2-(4-cyan-3-methyl-phenyl)-ethan
1-[p-(trans-4-Pentylcyclohexyl)-phenyl]-2-(4-cyan-3-methyl-phenyl)-ethan
1-[p-(trans-4-Heptylcyclohexyl)-phenyl]-2-(4-cyan-3-methyl-phenyl)-ethan
1-[p-(trans-4-Ethylcyclohexyl)-phenyl]-2-(4-cyan-2-fluor-phenyl)-ethan
1-[p-(trans-4-Propylcyclohexyl)-phenyl]-2-(4-cyan-2-fluor-phenyl)-ethan
1-[p-(trans-4-Butylcyclohexyl)-phenyl]-2-(4-cyan-2-fluor-phenyl)-ethan
1-[p-(trans-4-Pentylcyclohexyl)-phenyl]-2-(4-cyan-2-fluor-phenyl)-ethan
1-[p-(trans-4-Heptylcyclohexyl)-phenyl]-2-(4-cyan-2-fluor-phenyl)-ethan
1-[4-(trans-4-Ethylcyclohexyl)-2-fluorphenyl]-2-(4-cyan-phenyl)-ethan
1-[4-(trans-4-Propylcyclohexyl)-2-fluorphenyl]-2-(4-cyan-phenyl)-ethan
1-[4-(trans-4-Butylcyclohexyl)-2-fluorphenyl]-2-(4-cyan-phenyl)-ethan
1-[4-(trans-4-Pentylcyclohexyl)-2-flurophenyl]-2-(4-cyan-phenyl)-ethan
1-[4-(trans-4-Heptylcyclohexyl)-2-fluorphenyl]-2-(4-cyan-phenyl)-ethan
1-[4-(trans-4-Ethylcyclohexyl)-3-fluorphenyl]-2-(4-cyan-phenyl)-ethan
1-[4-(trans-4-Propylcyclohexyl)-3-fluorphenyl]-2-(4-cyan-phenyl)-ethan
1-[4-(trans-4-Butylcyclohexyl)-3-fluorphenyl]-2-(4-cyan-phenyl)-ethan
1-[4-(trans-4-Pentylcyclohexyl)-3-fluorphenyl]-2-(4-cyan-phenyl)-ethan

1-[4-(trans-4-Heptylcyclohexyl)-3-fluorphenyl]-2-(4-cyan-phenyl)-ethan
1-[4-(trans-4-Ethylcyclohexyl)-2-methylphenyl]-2-(4-cyan-phenyl)-ethan
1-[4-(trans-4-Propylcyclohexyl)-2-methylphenyl]-2-(4-cyan-phenyl)-ethan
1-[4-(trans-4-Butylcyclohexyl)-2-methylphenyl]-2-(4-cyan-phenyl)-ethan
1-[4-(trans-4-Pentylcyclohexyl)-2-methylphenyl]-2-(4-cyan-phenyl)-ethan
1-[4-(trans-4-Heptylcyclohexyl)-2-methylphenyl]-2-(4-cyan-phenyl)-ethan
1-[4-(trans-4-Ethylcyclohexyl)-2-chlorphenyl]-2-(4-cyan-phenyl)-ethan
1-[4-(trans-4-Propylcyclohexyl)-2-chlorphenyl]-2-(4-cyan-phenyl)-ethan
1-[4-(trans-4-Butylcyclohexyl)-2-chlorphenyl]-2-(4-cyan-phenyl)-ethan
1-[4-(trans-4-Pentylcyclohexyl)-2-chlorphenyl]-2-(4-cyan-phenyl)-ethan
1-[4-(trans-4-Heptylcyclohexyl)-2-chlorphenyl]-2-(4-cyan-phenyl)-ethan

## Beispiel 2

Ein Gemisch aus 38,4 g 1-(trans-4-n-Propylcyclohexyl)-2-(4'-brom-2'-fluorbiphenyl-4-yl)-ethan (F. 61,1°, K. 114,7°), 10 g $Cu_2$ $(CN)_2$, 120 ml Pyridin und 60 ml N-Methylpyrrolidon wird 2 Stunden auf 160° erhitzt. Man kühlt ab, gibt eine Lösung von 120 g $FeCl_3$ . $6H_2O$ in 600 ml 20 %iger Salzsäure hinzu, erwärmt 1,5 Stunden unter Rühren auf 70°, arbeitet wie üblich auf und erhält 1-(trans-4-n-Propylcyclohexyl)-2-(4'-cyan-2'-fluorbiphenyl-4-yl)-ethan, F. 89,4°, K. 168,4°.

## Beispiel 3

Analog Beispiel 2 erhält man aus 1-(trans-4-n-Pentylcyclohexyl)-2-(4'-brom-2'-fluorbiphenyl-4-yl-ethan (F. 57,5°, K. 113,7°) 1-(trans-4-n-Pentylcyclohexyl)-2-(4'-cyan-2'-fluorbiphenyl-4-yl)-ethan, F. 76,0°, K. 165,6°.

## Beispiel 4

Analog Beispiel 2, erhält man aus 1-(trans-4-n-Heptylcyclohexyl)-2-(4'-brom-2'-fluorbiphenyl-4-yl)-ethan (F. 54,0°, K. 113,0°) 1-(trans-4-n-Heptylcyclohexyl)-2-(4'-cyan-2'-fluorbiphenyl-4-yl)-ethan, F. 74,5°, K. 157,4°, $S_A$/N 108,0°.
Analog werden hergestellt:

1-(trans-4-Methylcyclohexyl)-2-(4'-cyan-2'-fluorbiphenyl-4-yl)-ethan
1-(trans-4-Ethylcyclohexyl)-2-(4'-cyan-2'-fluorbiphenyl-4-yl)-ethan
1-(trans-4-Butylcyclohexyl)-2-(4'-cyan-2'-fluorbiphenyl-4-yl)-ethan
1-(trans-4-Octylcyclohexyl)-2-(4'-cyan-2'-fluorbiphenyl-4-yl)-ethan
1-(trans-4-Nonylcyclohexyl)-2-(4'-cyan-2'-fluorbiphenyl-4-yl)-ethan
1-(trans-4-Decylcyclohexyl)-2-(4'-cyan-2'-fluorbiphenyl-4-yl)-ethan
1-(trans-4-Methylcyclohexyl)-2-(4'-cyan-2-fluorbiphenyl-4-yl)-ethan
1-(trans-4-Ethylcyclohexyl)-2-(4'-cyan-2-fluorbiphenyl-4-yl)-ethan
1-(trans-4-Propylcyclohexyl)-2-(4'-cyan-2-fluorbiphenyl-4-yl)-ethan, F. 65,4°, K. 161,3°
1-(trans-4-Butylcyclohexyl)-2-(4'-cyan-2-fluorbiphenyl-4-yl)-ethan
1-(trans-4-Pentylcyclohexyl)-2-(4'-cyan-2-fluorbiphenyl-4-yl)-ethan, F. 71,4°, K. 157,3°
1-(trans-4-Heptylcyclohexyl)-2-(4'-cyan-2-fluorbiphenyl-4-yl)-ethan, F. 72,9°, K. 149,5°
1-(trans-4-Octylcyclohexyl)-2-(4'-cyan-2-fluorbiphenyl-4-yl)-ethan
1-(trans-4-Nonylcyclohexyl)-2-(4'-cyan-2-fluorbiphenyl-4-yl)-ethan
1-(trans-4-Decylcyclohexyl)-2-(4'-cyan-2-fluorbiphenyl-4-yl)-ethan
1-(trans-4-Ethylcyclohexyl)-2-(4'-cyan-2-methylbiphenyl-4-yl)-ethan
1-(trans-4-Propylcyclohexyl)-2-(4'-cyan-2-methylbiphenyl-4-yl)-ethan
1-(trans-4-Butylcyclohexyl)-2-(4'-cyan-2-methylbiphenyl-4-yl)-ethan
1-(trans-4-Pentylcyclohexyl)-2-(4'-cyan-2-methylbiphenyl-4-yl)-ethan
1-(trans-4-Heptylcyclohexyl)-2-(4'-cyan-2-methylbiphenyl-4-yl)-ethan

## Beispiel 5

Ein Gemisch aus 43,0 g 1-(trans-4-n-Pentylcyclohexyl)-2-(4'-brom-3'-fluorbiphenyl-4-yl)-ethan (F. 62,3°, K. 123,2°), 10 g $Cu_2(CN)_2$, 120 ml Pyridin und 60 ml N-Methylpyrrolidon wird 2 Stunden auf 160° erhitzt und analog Beispiel 2 weiterverarbeitet. Man erhält 1-(trans-4-n-Pentylcyclohexyl)-2-(4'-cyan-3'-fluorbiphenyl-4-yl)-ethan, F. 58,5°, K. 149,7°.
Analog werden hergestellt:

1-(trans-4-Methylcyclohexyl)-2-(4'-cyan-3'-fluorbiphenyl-4-yl)-ethan
1-(trans-4-Ethylcyclohexyl)-2-(4'-cyan-3'-fluorbiphenyl-4-yl)-ethan
1-(trans-4-Propylcyclohexyl)-2-(4'-cyan-3'-fluorbiphenyl-4-yl)-ethan
1-(trans-4-Butylcyclohexyl)-2-(4'-cyan-3'-fluorbiphenyl-4-yl)-ethan

1-(trans-4-Heptylcyclohexyl)-2-(4′-cyan-3′-fluorbiphenyl-4-yl)-ethan
1-(trans-4-Octylcyclohexyl)-2-(4′-cyan-3′-fluorbiphenyl-4-yl)-ethan
1-(trans-4-Nonylcyclohexyl)-2-(4′-cyan-3′-fluorbiphenyl-4-yl)-ethan
1-(trans-4-Decylcyclohexyl)-2-(4′-cyan-3′-fluorbiphenyl-4-yl)-ethan
1-[4-(trans-4-Ethylcyclohexyl)-phenyl]-2-(4′-cyan-2-fluorbiphenyl-4-yl)-ethan
1-[4-(trans-4-Propylcyclohexyl)-phenyl]-2-(4′-cyan-2-fluorbiphenyl-4-yl)-ethan
1-[4-(trans-4-Butylcyclohexyl)-phenyl]-2-(4′-cyan-2-fluorbiphenyl-4-yl)-ethan
1-[4-(trans-4-Pentylcyclohexyl)-phenyl]-2-(4′-cyan-2-fluorbiphenyl-4-yl)-ethan
1-[4-(trans-4-Heptylcyclohexyl)-phenyl]-2-(4′-cyan-2-fluorbiphenyl-4-yl)-ethan
1-[4-(trans-4-Ethylcyclohexyl)-phenyl]-2-(4′-cyan-2′-fluorbiphenyl-4-yl)-ethan
1-[4-(trans-4-Propylcyclohexyl)-phenyl]-2-(4′-cyan-2′-fluorbiphenyl-4-yl)-ethan
1-[4-(trans-4-Butylcyclohexyl)-phenyl]-2-(4′-cyan-2′-fluorbiphenyl-4-yl)-ethan
1-[4-(trans-4-Pentylcyclohexyl)-phenyl]-2-(4′-cyan-2′-fluorbiphenyl-4-yl)-ethan
1-[4-(trans-4-Heptylcyclohexyl)-phenyl]-2-(4′-cyan-2′-fluorbiphenyl-4-yl)-ethan
1-[4-(trans-4-Ethylcyclohexyl)-phenyl]-2-(4′-cyan-2′-chlorbiphenyl-4-yl)-ethan
1-[4-(trans-4-Propylcyclohexyl)-phenyl]-2-(4′-cyan-2′-chlorbiphenyl-4-yl)-ethan
1-[4-(trans-4-Butylcyclohexyl)-phenyl]-2-(4′-cyan-2′-chlorbiphenyl-4-yl)-ethan
1-[4-(trans-4-Pentylcyclohexyl)-phenyl]-2-(4′-cyan-2′-chlorbiphenyl-4-yl)-ethan
1-[4-(trans-4-Heptylcyclohexyl)-phenyl]-2-(4′-cyan-2′-chlorbiphenyl-4-yl)-ethan
1-[4-(trans-4-Ethylcyclohexyl)-phenyl]-2-(4′-cyan-2′-methylbiphenyl-4-yl)-ethan
1-[4-(trans-4-Propylcyclohexyl)-phenyl]-2-(4′-cyan-2′-methylbiphenyl-4-yl)-ethan
1-[4-(trans-4-Butylcyclohexyl)-phenyl]-2-(4′-cyan-2′-methylbiphenyl-4-yl)-ethan
1-[4-(trans-4-Pentylcyclohexyl)-phenyl]-2-(4′-cyan-2′-methylbiphenyl-4-yl)-ethan
1-[4-(trans-4-Heptylcyclohexyl)-phenyl]-2-(4′-cyan-2′-methylbiphenyl-4-yl)-ethan
1-(trans, trans-4-Ethylbicylohex-4′-yl)-2-(4′-cyan-2-fluor-biphenyl-4-yl)-ethan
1-(trans, trans-4-Propylbicylohex-4′-yl)-2-(4′-cyan-2-fluor-biphenyl-4-yl)-ethan
1-(trans, trans-4-Butylbicylohex-4′-yl)-2-(4′-cyan-2-fluor-biphenyl-4-yl)-ethan
1-(trans, trans-4-Pentylbicylohex-4′-yl)-2-(4′-cyan-2-fluor-biphenyl-4-yl)-ethan
1-(trans, trans-4-Heptylbicylohex-4′-yl)-2-(4′-cyan-2-fluor-biphenyl-4-yl)-ethan
1-(trans, trans-4-Ethylbicylohex-4′-yl)-2-(4′-cyan-2′-fluor-biphenyl-4-yl)-ethan
1-(trans, trans-4-Propylbicylohex-4′-yl)-2-(4′-cyan-2′-fluor-biphenyl-4-yl)-ethan
1-(trans, trans-4-Butylbicylohex-4′-yl)-2-(4′-cyan-2′-fluor-biphenyl-4-yl)-ethan
1-(trans, trans-4-Pentylbicylohex-4′-yl)-2-(4′-cyan-2′-fluor-biphenyl-4-yl)-ethan
1-(trans, trans-4-Heptylbicylohex-4′-yl)-2-(4′-cyan-2′-fluor-biphenyl-4-yl)-ethan
1-(trans, trans-4-Ethylbicylohex-4′-yl)-2-(4′-cyan-2′-chlor-biphenyl-4-yl)-ethan
1-(trans, trans-4-Propylbicylohex-4′-yl)-2-(4′-cyan-2′-chlor-biphenyl-4-yl)-ethan
1-(trans, trans-4-Butylbicylohex-4′-yl)-2-(4′-cyan-2′-chlor-biphenyl-4-yl)-ethan
1-(trans, trans-4-Pentylbicylohex-4′-yl)-2-(4′-cyan-2′-chlor-biphenyl-4-yl)-ethan
1-(trans, trans-4-Heptylbicylohex-4′-yl)-2-(4′-cyan-2′-chlor-biphenyl-4-yl)-ethan
1-(trans, trans-4-Ethylbicylohex-4′-yl)-2-(4′-cyan-2′-methyl-biphenyl-4-yl)-ethan
1-(trans, trans-4-Propylbicylohex-4′-yl)-2-(4′-cyan-2′-methyl-biphenyl-4-yl)-ethan
1-(trans, trans-4-Butylbicylohex-4′-yl)-2-(4′-cyan-2′-methyl-biphenyl-4-yl)-ethan
1-(trans, trans-4-Pentylbicylohex-4′-yl)-2-(4′-cyan-2′-methyl-biphenyl-4-yl)-ethan
1-(trans, trans-4-Heptylbicylohex-4′-yl)-2-(4′-cyan-2′-methyl-biphenyl-4-yl)-ethan

## Beispiel 6

Ein Gemisch aus 3,6 g (trans-4-n-Pentylcyclohexyl)-ethylmalondialdehyd-bis-diethylacetal und 2,1 g des Carboximidamids des 4-Cyan-2-fluor-benzoesäureethyl-esters wird 20 Stunden auf 145° erhitzt. Nach dem Abkühlen wird der Rückstand in Ethanol gelöst, wie üblich aufgearbeitet und der erhaltene Ester wie üblich in das Nitril übergeführt. Man erhält 2-(4-Cyan-3-fluorphenyl)-5-[2-(trans-4-n-pentylcyclohexyl)-ethyl]-pyrimidin.

Analog werden hergestellt :

2-(4-Cyan-3-fluorphenyl)-5-[2-(trans-4-ethylcyclohexyl)-ethyl]-pyrimidin
2-(4-Cyan-3-fluorphenyl)-5-[2-(trans-4-propylcyclohexyl)-ethyl]-pyrimidin
2-(4-Cyan-3-fluorphenyl)-5-[2-(trans-4-butylcyclohexyl)-ethyl]-pyrimidin
2-(4-Cyan-3-fluorphenyl)-5-[2-(trans-4-heptylcyclohexyl)-ethyl]-pyrimidin
2-(4-Cyan-3-methylphenyl)-5-[2-(trans-4-ethylcyclohexyl)-ethyl]-pyrimidin
2-(4-Cyan-3-methylphenyl)-5-[2-(trans-4-propylcyclohexyl)-ethyl]-pyrimidin
2-(4-Cyan-3-methylphenyl)-5-[2-(trans-4-butylcyclohexyl)-ethyl]-pyrimidin
2-(4-Cyan-3-methylphenyl)-5-[2-(trans-4-pentylcyclohexyl)-ethyl]-pyrimidin
2-(4-Cyan-3-methylphenyl)-5-[2-(trans-4-heptylcyclohexyl)-ethyl]-pyrimidin
2-(4-Cyanphenyl)-5-[2-(trans-4-ethylcyclohexyl)-ethyl]-pyrimidin
2-(4-Cyanphenyl)-5-[2-(trans-4-propylcyclohexyl)-ethyl]-pyrimidin

2-(4-Cyanphenyl)-5-[2-(trans-4-butylcyclohexyl)-ethyl]-pyrimidin
2-(4-Cyanphenyl)-5-[2-(trans-4-pentylcyclohexyl)-ethyl]-pyrimidin
2-(4-Cyanphenyl)-5-[2-(trans-4-heptylcyclohexyl)-ethyl]-pyrimidin
2-(4-Cyanphenyl)-5-[2-(trans-4-ethylcyclohexyl)-ethyl]-pyrazin
2-(4-Cyanphenyl)-5-[2-(trans-4-propylcyclohexyl)-ethyl]-pyrazin
2-(4-Cyanphenyl)-5-[2-(trans-4-butylcyclohexyl)-ethyl]-pyrazin
2-(4-Cyanphenyl)-5-[2-(trans-4-pentylcyclohexyl)-ethyl]-pyrazin
2-(4-Cyanphenyl)-5-[2-(trans-4-heptylcyclohexyl)-ethyl]-pyrazin

Die folgenden Beispiele betreffen erfindungsgemäße flüssigkristalline Phasen :

## Beispiel A

Man stellt ein Gemisch her aus

15 % p-trans-4-Propylcyclohexyl-benzonitril,
27 % trans-1-p-Ethylphenyl-4-propylcyclohexan,
10 % trans-2-p-Ethoxyphenyl-4-propylcyclohexan,
7 % 4-Cyan-4'-(trans-4-pentylcyclohexyl)-biphenyl,
10 % 4-(trans-4-Pentylcyclohexyl)-4'-(trans-4-propylcyclohexyl)-biphenyl,
8 % p-trans-4-Propylcyclohexyl-benzoesäure-(p-propylphenylester),
6 % p-trans-4-Pentylcyclohexyl-benzoesäure-(p-propylphenylester) und
17 % 2-(4-Cyan-2-fluorphenyl)-5-[2-(trans-4-pentyl-cyclohexyl)-ethyl]-pyrimidin.

## Beispiel B

Man stellt ein Gemisch her aus

15 % p-trans-4-Propylcyclohexyl-benzonitril,
12 % p-trans-4-Butylcyclohexyl-benzonitril,
14 % trans-1-p-Ethylphenyl-4-propylcyclohexan,
10 % trans-1-p-Ethoxyphenyl-4-propylcyclohexan,
6 % 4-Cyan-4'-(trans-4-pentylcyclohexyl)-biphenyl,
4 % 4,4'-Bis-(trans-4-propylcyclohexyl)-biphenyl,
6 % 4-(trans-4-Pentylcyclohexyl)-4'-(trans-4-propylcyclohexyl)-biphenyl,
8 % p-trans-4-Propylcyclohexyl-benzoesäure-(p-propylphenylester),
16 % p-trans-4-Pentylcyclohexyl-benzoesäure-(p-propylphenylester) und
9 % 1-[4-(4-trans-Pentylcyclohexyl)-2-fluor-phenyl]-2-(4-cyanphenyl)-ethan.

## Beispiel C

Man stellt ein Gemisch her aus

19,0 % 4-(trans-4-n-Propylcyclohexyl)-benzonitril,
29,0 % 4-(trans-4-n-Pentylcyclohexyl)-benzonitril,
20,0 % 4-(trans-4-n-Heptylcyclohexyl)-benzonitril,
12,0 % trans-4-n-Pentyl-(4'-cyanobiphenylyl-4)-cyclohexan,
8,5 % 4,4'-Bis-(trans-4-propylcyclohexyl)-biphenyl
11,0 % 2-p-Cyanphenyl-4'-heptyl-1,3-dioxan und
0,5 % 1-[p-(4-trans-Butylcyclohexyl)-phenyl]-2-(4-cyan-2-fluorphenyl)-ethan.

## Beispiel D

Man stellt ein Gemisch her aus

19 % 4-(trans-4-n-Propylcyclohexyl)-benzonitril,
28 % 4-(trans-4-n-Pentylcyclohexyl)-benzonitril,
11 % trans-4-n-Pentyl(4'-cyanobiphenylyl-4)-cyclohexan,
10 % 4-(trans-4-n-Propylcyclohexyl)-benzoesäure-(4-propyl-phenyl)-ester,
12 % 4-(trans-4-n-Propylcyclohexyl)-benzoesäure-(trans-4-n-[propylcyclohexyl]-ester),
12 % 1-(4-trans-Pentylcyclohexyl)-2-(4'-cyan-2'-fluor-biphenyl-4-yl)-ethan,
7 % 2-(4-Cyanphenyl)-5-[2-(trans-4-pentylcyclohexyl)-ethyl]-pyrimidin und
1 % 1-[p-(4-trans-Pentylcyclohexyl)-phenyl]-2-(4'-cyan-2-fluorbiphenyl-4-yl)-ethan.

Beispiel E

Man stellt ein Gemisch her aus

3 % 4-Ethyl-4'-(trans-4-propylcyclohexyl)-biphenyl,
5 % 4-Ethyl-4'-(trans-4-pentylcyclohexyl)-biphenyl,
5 % 2-p-Pentyloxyphenyl-5-hexylpyrimidin,
5 % 2-p-Hexyloxyphenyl-5-hexylpyrimidin,
6 % 2-p-Heptyloxyphenyl-5-hexylpyrimidin,
8 % 2-p-Nonyloxyphenyl-5-hexylpyrimidin,
8 % 2-p-Undecyloxyphenyl-5-hexylpyrimidin,
7 % trans-4-Propylcyclohexancarbonsäure-(p-methoxyphenylester),
5 % trans-4-Butylcyclohexancarbonsäure-(p-methoxyphenylester),
4 % trans-4-Pentylcyclohexancarbonsäure-(p-methoxyphenylester),
18 % 2-(4-Cyan-2-fluorphenyl)-5-[2-(trans-4-pentyl-cyclohexyl)-ethyl]-pyrimidin und
12 % 2-(4-Cyanphenyl)-5-[2-(trans-4-propylcyclohexyl)-ethyl]-pyrimidin.
10 % trans-1-p-Ethylphenyl-4-pentylcyclohexan und
4 % Buttersäure-(p-trans-4-propylcyclohexyl-phenylester).

Diese Mischung ist gut geeignet als Dielektrikum für Flüssigkristallanzeigelemente mit hohem Multiplexverhältnis.

Beispiel F

Man stellt ein Gemisch her aus

15 % p-trans-4-Propylcyclohexyl-benzonitril,
27 % trans-1-p-Ethylphenyl-4-propylcyclohexan,
10 % trans-2-p-Ethoxyphenyl-4-propylcyclohexan,
7 % 4-Cyan-4'-(trans-4-pentylcyclohexyl)-biphenyl,
10 % 4-(trans-4-Pentylcyclohexyl)-4'-(trans-4-propylcyclohexyl)-biphenyl,
8 % p-trans-4-Propylcyclohexyl-benzoesäure-(p-propylphenylester),
6 % p-trans-4-Pentylcyclohexyl-benzoesäure-(p-propylphenylester),
8 % 1-(trans-4-Propylcyclohexyl)-2-(4'-cyan-2'-fluorbiphenyl-4-yl)-ethan,
5 % 1-(trans-4-Pentylcyclohexyl)-2-(4'-cyan-2'-fluorbiphenyl-4-yl)-ethan und
4 % 1-(trans-4-Heptylcyclohexyl)-2-(4'-cyan-2'-fluorbiphenyl-4-yl)-ethan.

**Patentansprüche**

1. Ethanderivate der Formel I

$$R^1—A^1—CH_2CH_2—A^2—(A^3)_n—R^2 \qquad I$$

worin
$R^1$ H, eine Alkylgruppe mit 1-12 C-Atomen, worin auch eine oder zwei nicht benachbarte $CH_2$-Gruppen durch O-Atome und/oder CO-Gruppen und/oder —O—CO-Gruppen und/oder —CO—O-Gruppen ersetzt sein können, F, Cl, Br
$R^2$ —CN oder —$Z^2$—$A^4$—CN
$A^1$ —A—, —A—$A^5$— oder —$A^5$—A—,
$A^2$ eine unsubstituierte oder durch ein oder zwei F- und/oder Cl-Atome und/oder $CH_3$-Gruppen und/oder CN-Gruppen substituierte 1,4-Phenylen-Gruppe, worin auch eine oder zwei CH-Gruppen durch N-Atome ersetzt sein können,
A eine 1,4-Cyclohexylen-Gruppe, worin auch eine oder zwei nicht benachbarte $CH^2$-Gruppen durch O-Atome ersetzt sein können, eine 1,3-Dithian-2,5-diylgruppe oder eine 1,4-Bicyclo(2,2,2)-octylen-Gruppe,
$A^3$, $A^4$ und $A^5$ jeweils eine 1,4-Cyclohexylengruppe, worin auch eine oder zwei nicht benachbarte $CH_2$-Gruppen durch O-Atome ersetzt sein können, eine 1,3-Dithian-2,5-diylgruppe, eine unsubstituierte oder durch ein oder zwei F- und/oder Cl-Atome und/oder $CH_3$-Gruppen und/oder CN-Gruppen substituierte 1,4-Phenylen-Gruppe, worin auch eine oder zwei CH-Gruppen durch N-Atome ersetzt sein können,
n 0 oder 1,
$Z^2$ —$CH_2CH_2$—, —$CH_2O$—, —$OCH_2$—, —C—CO— oder eine Einfachbindung, und
bedeutet, mit der Maßgabe, daß
a) im Falle $A^2$ = $A^3$ = 1,4-Phenylen (n = 1) und/oder im Falls $R^1$—$A^1$ = p-(trans-4-Alkylcyclohexyl)-phenyl mindestens eine der Gruppen $A^2$ bzw. $A^3$ lateral substituiert ist,
b) im Falle $A^1$ = A und/oder $R^1$—$A^1$ = trans-4-(trans-4-Alkylcyclohexyl)-cyclohexyl n 1 und $A^3$ eine

unsubstituierte oder durch ein oder zwei F- und/oder Cl-Atome und/oder $CH_3$-Gruppen und/oder CN-Gruppen substituierte 1,4-Phenylengruppe bedeutet, und

c) p-[2-[trans-4-(p-Alkylphenyl oder p-Alkoxyphenyl) cyclohexyl]-äthyl]benzonitrile ausgeschlossen sind.

2. Verfahren zur Herstellung von Ethanderivaten der Formel I, dadurch gekennzeichnet, daß man ein entsprechendes Carbonsäureamid dehydratisiert oder ein entsprechendes Carbonsäurehalogenid mit Sulfamid umsetzt, oder daß man eine entsprechende Brom- oder Chlorverbindung mit einem Cyanid umsetzt.

3. Verwendung der Ethanderivate der Formel I nach Anspruch 1 als Komponenten flüssigkristalliner Phasen.

4. Flüssigkristalline Phase mit mindestens zwei flüssigkristallinen Komponenten, dadurch gekennzeichnet, daß mindestens eine Komponente ein Ethanderivat der Formel I nach Anspruch 1 ist.

5. Flüssigkristallanzeigeelement, dadurch gekennzeichnet, daß es eine Phase nach Anspruch 4 enthält.

6. Elektrooptisches Anzeigeelement nach Anspruch 5, dadurch gekennzeichnet, daß es als Dielektrikum eine Phase nach Anspruch 4 enthält.

**Claims**

1. Ethane derivatives of the formula I

$$R^1—A^1—CH_2CH_2—A^2—(A^3)_n—R^2 \qquad I$$

wherein

$R^1$ is H, an alkyl group with 1-12 C atoms, in which one or two $CH_2$ non adjacent groups can also be replaced by O atoms and/or CO-groups and/or —O—CO-groups and/or —CO—O-groups, F, C1, Br,

$R^2$ is —CN or $—Z^2—A^4—CN$,

$A^1$ is —A—, $—A—A^5—$ or $—A^5—A—$,

$A^2$ is a 1,4-phenylene group which is unsubstituted or substituted by one or two F and/or Cl atoms and/or $CH_3$ groups and/or CN goups ans in which one or two CH groups can also be replaced by N atoms,

A is a 1,4-cyclohexylene group, in which one or two non adjacent $CH_2$ groups can also be replaced by O atoms, or is a 1,3-dithiane-2,5-diyl group or 1,4-bicyclo(2,2,2)-octylene group,

$A^3$, $A^4$ and $A^5$ are each a 1,4-cyclohexylene group, in which one or two non adjacent $CH_2$ groups can also be replaced by O atoms, or are each a 1,3-dithiane-2,5-diyl group, or a 1,4-phenylene group which is unsubstituted or substituted by one or two F and/or Cl atoms and/or $CH_3$ groups and/or CN groups, and in which one or two CH groups can also be replaced by N atoms,

n is 0 or 1,

$Z^2$ is $—CH_2CH_2—$, $—CH_2O—$, $—OCH_2—$, —O—CO— or a single bond and with the proviso that

a) if $A^2 = A^3 =$ 1,4-phenylene ($n = 1$) and/or if $R^1—A^1 =$ p-(trans-4-alkylcyclohexyl)-phenyl, at least one of the groups $A^2$ and/or $A^3$ is laterally substituted,

b) if $A^1 = A$ and/or $R^1—A^1 =$ trans-4-(trans-4-alkylcyclohexyl)-cyclohexyl, n is 1 and $A^3$ is a 1,4-phenylene group which is unsubstituted or substituted by one or two F and/or Cl atoms and/or $CH_3$ groups and/or CN groups, and

c) p-[2-[trans-4-(p-alkylphenyl or p-alkoxyphenyl) cyclohexyl]-ethyl]benzonitriles are excluded.

2. Process for the preparation of ethane derivatives of the formula I, characterised in that a corresponding carboxylic acid amide is dehydrated or a corresponding carboxylic acid halide is reacted with sulfamide, or in that a corresponding bromine or chlorine compound is reacted with a cyanide.

3. Use of the ethane derivatives of the formula I according to Claim 1 as components of liquid crystal phases.

4. Liquid crystal phase with at least two liquid crystal components, characterised in that as least one component is an ethane derivative of the formula I according to Claim 1.

5. Liquid crystal display element, characterised in that it contains a phase according to Claim 4.

6. Electrooptical display element according to Claim 5, characterised in that it contains a phase according to Claim 4 as a dielectric.

**Revendications**

1. La présente invention concerne des dérivés d'éthane de formule I

$$R^1—A^1—CH_2CH_2—A^2(A^3)_n—R^2 \qquad (I)$$

où

$R^1$ représente H, un groupe alcoyle en $C_1$-$C_{12}$, où encore un ou deux groupes $CH_2$ non voisins

peuvent être remplacés par des atomes d'oxygène et/ou des groupes CO et/ou des groupes —O—CO- et/ou des groupes —CO—O, F, Cl, Br

$R^2$ représente —CN ou —$Z^2$—$A^4$—CN

$A^1$ représente —A—, —A—$A^5$, ou —$A^5$—A—,

$A^2$ représente un groupe 1,4-phénylène non substitué ou substitué par un ou deux atomes de F et/ou Cl et/ou groupe $CH_3$ et/ou groupe CN, où encore un ou deux groupes CH peuvent être remplacés par des atomes de N,

A représente un groupe 1,4-cyclohexylène, où encore un ou deux groupes $CH_2$ non voisins peuvent être remplacés par des atomes d'oxygène, un groupe 1,3-dithian-2,5-diyle ou un groupe 1,4-bicyclo - 2,2,2)-octylène,

$A^3$, $A^4$ et $A^5$ représentent chacun un groupe 1,4-cyclo-hexylène où encore un ou deux groupes $CH_2$ non voisins peuvent être remplacés par des atomes d'oxygène, un groupe 1,3-dithian-2,5-diyle, un groupe 1,4-phénylène non substitué ou substitué par un ou deux atomes de F et/ou Cl et/ou groupe $CH_3$ et/ou groupe CN, où encore un ou deux groupes CH peuvent être remplacés par des atomes de N,

n vaut 0 ou 1,

$Z^2$ représente —$OH_2CH_2$—, —$CH_2O$—, —$OCH_2$—, —O—CO ou une liaison simple, et avec la précision que :

a) dans le cas où $A^2$ = $A^3$ = 1,4-phénylène (n = 1) et/ou dans le cas où $R^1$—$A^1$ = p-(trans-4-alcoylcyclohexyl)-phényle au moins l'un des groupes $A^2$ ou $A^3$ est substitué latéralement, et

b) dans le cas où $A^1$ = A et/ou $R^1$—$A^1$ = (trans-4-(trans-alcoylcyclohexyl)-cyclohexyle, n vaut 1 et $A^3$ représente un groupe 1,4-phénylène non substitué ou substitué par un ou deux atomes de F et/ou Cl et/ou groupes $CH_3$ et/ou groupes CN et

c) p-[2-[trans-4-alcoylphényle ou p-alcoxyphényl) cyclohexyl]-ethyl]benzonitriles, étant exclus.

2. Procédé de préparation de dérivés d'éthane de formule I, caractérisé en ce qu'on déshydrate un amide d'acide carboxylique correspondant ou en ce qu'on fait réagir un halogénure d'acide carboxylique correspondant avec un sulfamide, ou en ce qu'on fait réagir un composé de brome ou chlore correspondant avec un cyanure.

3. Application des dérivés d'éthane de formule I selon la revendication I comme composants de phases à cristaux liquides.

4. Phase à cristaux liquides ayant au moins deux composants à cristaux liquides, caractérisée en ce qu'au moins un composant est un dérivé d'éthane de formule I selon la revendication I.

5. Elément d'affichage à cristaux liquides caractérisé en ce qu'il contient une phase selon la revendication 4.

6. Elément d'affichage électro-optique selon la revendication 5, caractérisé en ce qu'il contient comme diélectrique une phase selon la revendication 4.